# EUROPEAN PATENT SPECIFICATION

(11) **EP 3 903 741 B1**
(45) Date of publication and mention of the grant of the patent: **19.07.2023**
(21) Application number: 19905352.1
(22) Date of filing: 24.12.2019
(51) Int. Cl.: A61F 2/82

(54) **BIO-COATING AND IMPLANT**
BIO-BESCHICHTUNG UND IMPLANTAT
REVÊTEMENT BIOLOGIQUE ET IMPLANT

(30) Priority: 29.12.2018 CN 201811642573
(43) Date of publication of application: 03.11.2021
(73) Proprietor: Suzhou Microport Orthorecon Co., Ltd., Suzhou, Jiangsu 215000 (CN)
(72) Inventor: XIN, Chen, Shanghai 201203 (CN); LU, Lingxiao, Shanghai 201203 (CN); CHANG, Xiaolong, Shanghai 201203 (CN); LIU, Xiang, Shanghai 201203 (CN); YUE, Chengyun, Shanghai 201203 (CN)
(74) Representative: Lavoix
(86) International application number: PCT/CN2019/127936
(87) International publication number: WO 2020/135423

(56) References cited:
- US-A- 4 542 539
- US-A1- 2010 137 990
- US-A1- 2015 258 735

## Description

### TECHNICAL FIELD

The present application relates to the field of medical devices and, in particular, to a biological coating and an implant.

### BACKGROUND

Biological coatings for conventional implants are usually manufactured using techniques such as sintering of titanium beads, plasma spraying and corundum blasting. However, it is difficult for these techniques to change porosity of the biological coating in a controllable manner. Moreover, these techniques are unable to ensure connectivity between pores, which easily results in stress shielding. Furthermore, in such biological coatings, layer-to-layer and coating-to-substrate bonding is often accomplished by adhesives and therefore lack of stability, which is detrimental to bone ingrowth and to the mechanical properties of the biological coatings and implants. The conventional techniques also have drawbacks including high complexity, long production cycle and high cost.

There has been disclosed a 3D printed porous metal bone tissue scaffold with a changing pore size in gradient. This scaffold is a hexahedral structure consisting of densely arranged components A, B and C. The components A are arranged in a matrix as the outermost layer of the hexahedral structure. The components B are arranged in a matrix as the intermediate layer of the hexahedral structure. The components C are arranged in a matrix as the innermost layer of the hexahedral structure. The component A has a pore size greater than the component B which in turn has a pore size greater than the component C. However, the scaffold still has a single structure, because each of the components A, B and C has a hexahedral structure and is arranged within the scaffold in a regular and ordered way. Such scaffold has a distinctly different structure from the bone trabecular that has an irregular porous network structure with connected pores, resulting in a poor long-term stability and a limited bone ingrowth performance.

US 2015/258735 A1 describes a laser-produced porous surface. The obtained surface layer comprises a plurality of first monomers (unit cell) connected in an unordered manner; the plurality of first pores are formed between the plurality of first monomers and within an interior of the plurality of first monomers.

US 4 542 539 A describes a surgical implant having a graded porous coating.

### SUMMARY

An object of present application is to provide a biological coating and an implant to solve the problem that existing biological coatings and implants have a poor long-term stability and a limited bone ingrowth performance.

To solve the above problem, present application provides a biological coating according to claim 1.

Additional features of the biological coating are described in claims 2 to 9.

The present application also provides an implant according to claims 10 or 11.

The biological coating and implant provided by present application offer the following advantages:
Firstly, the surface layer is disposed on an outermost side of the biological coating and includes a plurality of first monomers connected in an unordered manner, a plurality of first pores formed between the plurality of first monomers and within the interior of the plurality of first monomers. The unordered connections between the first monomers in the surface layer allows the plurality of first pores in the surface layer being in an unordered arrangement, which facilitates cells of corresponding bone tissues to grow into the surface layer, thus allowing an improved bone ingrowth performance and a long-term stability.
Secondly, the biological coating further includes at least one intermediate layer, and the surface layer and the at least one intermediate layer are arranged in sequence along a direction from an outer side of the biological coating to an inner side of the biological coating, the porosity of the biological coating decreasing gradually along the direction from the outer side of the biological coating to the inner side of the biological coating. In this way, the surface layer of the biological coating is able to own a good bone ingrowth performance and a long-term stability, and the intermediate layer of the biological coating is able to own a good mechanical property, which enables to offer an overall stability to the biological coating.

In an embodiment of present application, due to the ordered connection between the plurality of second monomers in the intermediate layer, the plurality of second pores in the intermediate layer are able to be in the ordered arrangement, which offers a good mechanical property to the intermediate layer and biological coating.

In another embodiment of present application, since each of the first and second monomers connected in the unordered manner, the first pores in the first monomers connected in the unordered manner and the second pores in the second monomers connected in the unordered manner form a structure that is very similar to native cancellous bone in natural three-dimensional structure and physiology functions, which allows to further improve microstructure and biomechanical properties of the biological coating.

In yet another embodiment of present application, as the first monomer in the surface layer has a different structure with respect to the second monomers in the intermediate layer, the surface layer has a different porosity and mechanical properties with respect to the intermediate layer. Compared to the biological coating having varied porosities through merely varied diameters of the first connecting rods in the first monomers and the second connecting rods in the second monomers and/ or varied densities of arrangements for the first connecting rods in the first monomers and the second connecting rods in the second monomers, such embodiment is able to avoid the easy breakage of the first or second connecting rods arising from the excessively small diameter and the very sparse arrangement. In addition, such embodiment is also able to avoid the problem of difficult powder removal arising from the excessively large diameter and the very dense arrangement of the connecting rods.

In other embodiments, porosities of the surface and intermediate layer continuously vary in gradient along the direction from the outer side of the biological coating to the inner side of the biological coating, which makes fracture between layers arising from discontinuous in properties (e.g., porosity, rod diameter, density, etc.) less prone. This results in even better mechanical properties for the intermediate layer, a good bone ingrowth performance of the surface layer. Moreover, as a good transition is presented among the surface layer and the intermediate layer, the biological coating is able to have a better stability.

### BRIEF DESCRIPTION OF THE DRAWINGS

Fig. 1 is a structural schematic of a biological coating according to a embodiment 1 of present application.
Fig. 2 is a schematic enlarged view of a portion of the biological coating of Fig. 1.
Fig. 3 is a schematic diagram of a rhombic dodecahedron according to the embodiment 1 of present application.
Fig. 4 schematically illustrates the variation of porosity in the biological coating according to embodiment 1 of present application.
Fig. 5 schematically illustrates the variation of porosity in a biological coating according to an embodiment of present application.
Fig. 6 schematically illustrates the variation of porosity in a biological coating according to yet another embodiment of present application.
Fig. 7 is a structural schematic of an implant according to embodiment 1 of present application.
Fig. 8 is a structural schematic of a biological coating according to embodiment 2 of present application.
Fig. 9 is a schematic enlarged view of a portion of the biological coating of Fig. 8.
Fig. 10 is a structural schematic of an implant according to embodiment 2 of present application.
Fig. 11 is a structural schematic of a biological coating according to embodiment 3 of present application.
Fig. 12 is a schematic enlarged view of a portion of the biological coating of Fig. 11.
Fig. 13 is a schematic diagram of a diamond structure according to embodiment 3 of present application.
Fig. 14 is a structural schematic of an implant according to embodiment 3 of present application.
Fig. 15 is a structural schematic of a biological coating according to embodiment 4 of present application.
Fig. 16 is a schematic enlarged view of a portion of the biological coating of Fig. 15.
Fig. 17 is a structural schematic of an implant according to embodiment 4 of present application.
Fig. 18 is a structural schematic of a biological coating according to embodiment 5 of present application.
Fig. 19 is a schematic enlarged view of a portion of the biological coating of Fig. 18.
Fig. 20 is a structural schematic of an implant according to embodiment 5 of present application.
Fig. 21 is a structural schematic of an icosahedron according to embodiment 6 of present application.

In the figures,
100, biological coating; 110, surface layer; 120, intermediate layer; 140, first connecting rod; 150, second connecting rod; and
200, substrate layer.

### DETAILED DESCRIPTION

The present application provides a biological coating comprising a surface layer including a plurality of monomers connected in an unordered manner, a plurality of pores formed between the plurality of first monomers and within the interior of the plurality of first monomers. The unordered connections between the first monomers in the surface layer allows the plurality of first pores in the surface layer being in an unordered arrangement, which facilitates cells of corresponding bone tissues to grow into the surface layer, thus allowing an improved bone ingrowth performance and a long-term stability.

Accordingly, the present application also provides an implant comprising a substrate layer and a biological coating provided on the substrate layer. The unordered arrangement of the plurality of pores in the surface layer of the biological coating enables to improve the bone ingrowth performance and long-term stability of the implant.

The biological coating and implant proposed in present application will be described below in greater detail with reference to the accompanying drawings and specific embodiments. Features and advantages of present application will be more apparent from the following description and the appended claims. Note that the figures are provided in a very simplified form and not necessarily drawn to exact scale, which are only intended to facilitate the explaining of embodiments in a convenient and clear way.

### Embodiment 1

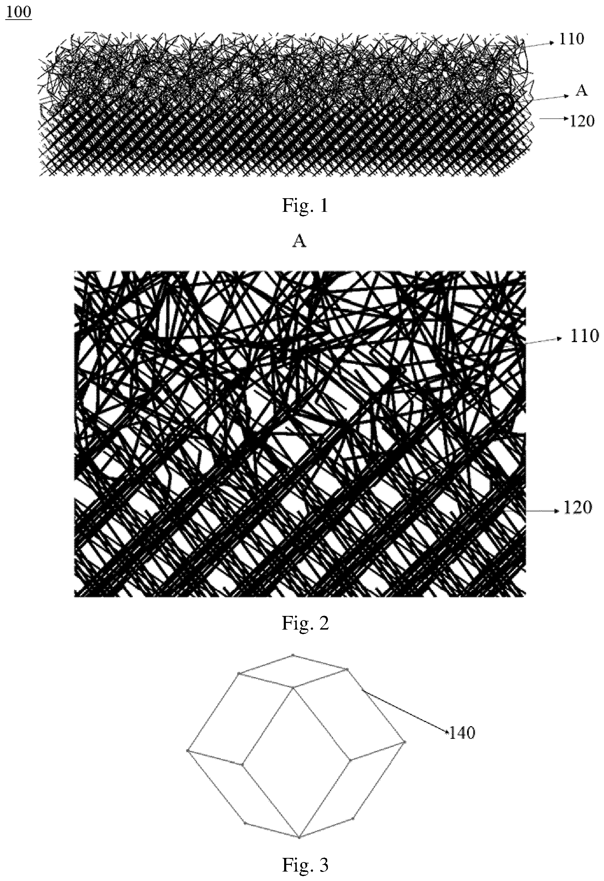

This embodiment provides a biological coating 100. Reference is now made to Fig. 1, a structural schematic of a biological coating 100 according to the embodiment 1of present application, and to Fig. 2, a schematic enlarged view of portion A of the biological coating 100 of Fig. 1. The biological coating 100 includes a surface layer 110 and an intermediate layer 120. The surface layer 110 is disposed on the intermediate layer 120. The surface layer 110 is disposed at the outermost layer of the biological coating 100. The surface layer 110 has a porosity greater than the intermediate layer 120.

The surface layer 110 includes a plurality of first monomers connected in an unordered manner. The plurality of first pores are formed between the plurality of first monomers and within the interior of the plurality of first monomers. Reference is now made to Fig. 3, a schematic of a rhombic dodecahedron according to embodiment 1 of present application. In this embodiment, the first monomer has a rhombic dodecahedral structure constructed from a plurality of first connecting rods 140 that connect to one another, which results in the formation of a plurality of first pores inside the first monomer. The first pores may also be defined by adjacent first monomers connected with each other.

The unordered connections between the plurality of first monomers in the surface layer 110 allows the plurality of first pores in the surface layer 110 being in an unordered arrangement, which facilitates cells of corresponding bone tissues to grow into the surface layer 110, thus allowing an improved bone ingrowth performance and a long-term stability.

The first pore may have a pore size of 100-1,000 µm, and the surface layer 110 may have a porosity of 50-80%. These allow biological coating 100 to have a good bone ingrowth performance and a long-term stability as well as allow the surface layer 110 to have a high roughness and a high coefficient of friction, which enables a good short-term stability of the biological coating 100.

The intermediate layer 120 includes a plurality of second monomers connected in an ordered manner. The plurality of second pores are formed between the plurality of second monomers and within the interior of the plurality of second monomers. In this embodiment, the second monomer has a rhombic dodecahedral structure constructed from a plurality of interconnected second connecting rods that connected to one another, which results in the formation of a plurality of second pores inside the second monomer. The second pores may also be defined by adjacent second monomers connected with each other.

Due to the ordered connection between the plurality of second monomers in the intermediate layer 120, the plurality of second pores in the intermediate layer 120 are able to be in the ordered arrangement, which offers a good mechanical property to the intermediate layer 120 and biological coating 100.

The second pore may have a pore size of 100-1,000 µm, and the intermediate layer 120 may have a porosity of 10-60%.

In this embodiment, the biological coating 100 may have a total thickness of 0.5-5.0 mm and a high porosity. The biological coating 100 may be designed with a computer software and integrally formed by a 3D printing. This allows the porosities of the surface and intermediate layers 110, 120 to vary in a controllable manner, resulting in a good interconnectivity between the first pores, between the second pores and among the first and second pores in the biological coating 100.

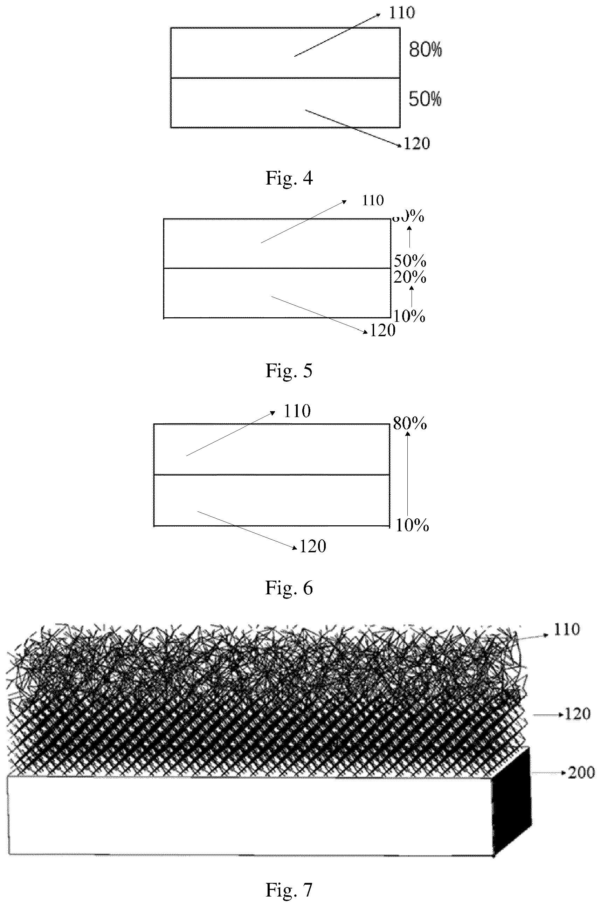

Reference is now made to Fig. 4, a schematic diagram illustrating the variation of the porosity in the biological coating 100 according to embodiment 1 of present application. The porosity of the intermediate layer 120 is lower than that of the surface layer 110. Each of the intermediate layer 120 and the surface layer 110 has a uniform porosity. That is, the porosity change occurs only among the intermediate layer 120 and the surface layer 110. The greater porosity of the surface layer 110 provides a sufficient ingrowth space for tissue cells and facilitates the transportation of nutrients and removal of metabolic wastes, enabling an improved bone ingrowth performance. The lower porosity of the intermediate layer 120 allows better mechanical properties.

The porosity change is able to result from a diameter and/or density variation of the first connecting rods 140 and the second connecting rods. Specifically, the first connecting rods 140 are smaller than the second connecting rods in diameter, the first connecting rods 140 in the surface layer 110 having an identical diameter, the second connecting rods in the intermediate layer 120 having an identical diameter; and/or the first connecting rods 140 are arranged in the surface layer 110 with a smaller density compared to the second connecting rods in the intermediate layer 120, the first connecting rods 140 in the surface layer 110 being arranged with uniform density, the second connecting rods in the intermediate layer 120 being arranged with uniform density.

In other embodiments, the biological coating 100 may have a different porosity variation from this embodiment.

Reference is now made to Fig. 5, a schematic illustration of a porosity variation of the biological coating 100 according to an embodiment of present application. Porosity of the intermediate layer 120 is lower than that of the surface layer 110, and in each single layer of the intermediate layer 120 and the surface layer 110, the porosity gradually decreases from outer side to inner side, where the side of the surface layer 110 away from the intermediate layer 120 is the outer side of the biological coating 100 and the side of the intermediate layer 120 away from the surface layer 110 is the inner side of the biological coating 100. The larger porosity of the surface layer 110 provides a sufficient ingrowth space for tissue cells and facilitates the transportation of nutrients and removal of metabolic wastes, enabling an improved bone ingrowth performance. The lower porosity of the intermediate layer 120 imparts better mechanical properties. The first connecting rods 140 in the surface layer 110 have a diameter gradually increasing along the direction from the outer side of the biological coating 100 to the inner side of the biological coating 100, the second connecting rods in the intermediate layer 120 having a diameter gradually increasing along the direction from the outer side of the biological coating 100 to the inner side of the biological coating 100, the first connecting rods 140 in the surface layer 110 being smaller than the second connecting rods in the intermediate layer 120 in diameter; and/or the first connecting rods 140 in the surface layer 110 are arranged in a density gradually increasing along the direction from the outer side of the biological coating 100 to the inner side of the biological coating 100, the second connecting rods in the intermediate layer 120 are arranged in a density gradually increasing along the direction from the outer side of the biological coating 100 to the inner side of the biological coating 100, and the first connecting rods 140 in the surface layer 110 are arranged in a smaller density compared to the second connecting rods in the intermediate layer 120.

With reference to Fig. 6, a schematic illustration of a porosity variation of the biological coating 100 according to another embodiment of present application, the porosity of intermediate layer 120 is smaller than that of the surface layer 110, and the porosity of the entire biological coating 100 gradually decreases from outer side to inner side. Preferably, the biological coating 100 has a porosity continuously changing in gradient along the direction from the outer side to the inner side, where the continuously changing in gradient indicates the linear variation of the porosity. Compared to the gradient change containing leaps (for example, the porosity only gradually decreases in each single layer) that tends to fracture at the interface between the layers due to stress concentration, the continuous change in gradient is able to avoid significant stress concentration to cause breakage between layers. Therefore, the intermediate layer 120 has enhanced mechanical properties and the surface layer 110 has an improved bone ingrowth performance. In addition, due to the smooth transition between the intermediate layer 120 and the surface layer 110, the biological coating 100 is able to possess a higher stability. Further, the biological coating 100 has diameters of the first connecting rods 140 and the second connecting rods gradually increasing along the direction from the outer side of the biological coating 100 to the inner side of the biological coating 100, and/or the biological coating 100 has the first connecting rods 140 and the second connecting rods arranged in a density gradually increasing along the direction from the outer side of the biological coating 100 to the inner side of the biological coating 100.

In one Embodiment, the at least one intermediate layer 120 has at least two intermediate layers. The surface layer 110 and the plurality of the intermediate layers 120 are arranged in sequence along the direction from the outer side of the biological coating to the inner side of the biological coating. Porosities of the intermediate layers 120 and the surface layer 110 in the biological coating 100 are uniform and invariable within each single layer and gradually decrease among different layers along the direction from the outer side to the inner side.

In another embodiment, each of the or more intermediate layers 120 has a porosity gradually decreases along the direction from the outer side to the inner side, and/or the surface layer 110 has a porosity gradually decreases along the direction from the outer side to the inner side.

In yet another embodiment, each of the plurality of intermediate layers 120 has a porosity lower than the surface layer 110, and the biological coating 100 has a porosity gradually decreases along the direction from the outer side to the inner side

In the above embodiments, the biological coating 100 has a structure similar to bone trabeculae.

This embodiment also provides an implant. Referring to Fig. 7, a structural schematic of an implant according to embodiment 1 of present application, the plant includes a substrate layer 200 and the biological coating 100 according to embodiment 1. The intermediate layer 120 in the biological coating is disposed on the substrate layer 200, with the surface layer 110 arranged at the outermost side of the implant. The substrate layer 200 may also be a porous coating layer, which has a porosity lower than the intermediate layer 120.

The substrate layer 200 may be integrally formed by a 3D print. Alternatively, the entirety of the implant is integrally formed by a 3D print.

### Embodiment 2

This embodiment provides a biological coating 100. The biological coating 100 in this embodiment differs from the biological coating 100 in embodiment 1 in that the plurality of second monomers in the intermediate layer 120 are connected in a unordered manner.

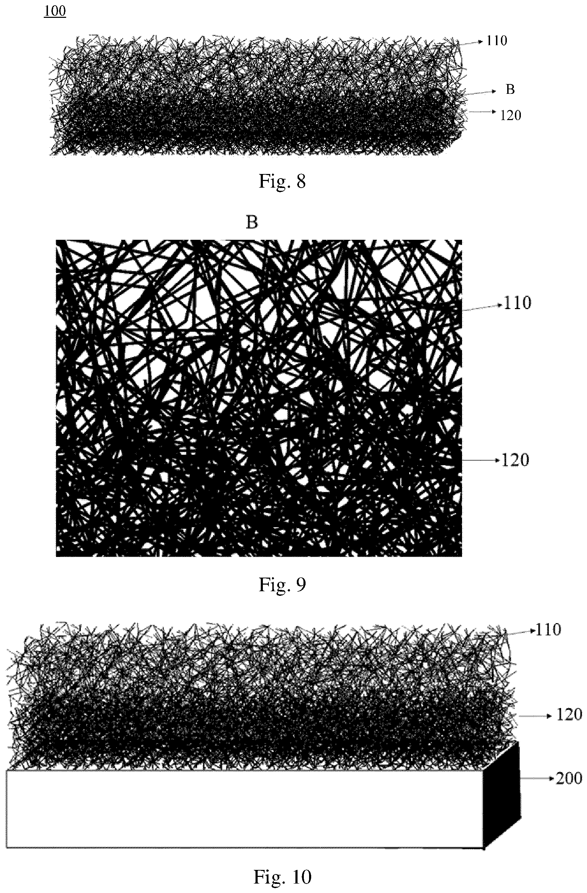

Reference is now made to Fig. 8, a structural schematic of the biological coating 100 according to embodiment 2 of present application, and to Fig. 9, a schematic enlarged view of portion B of the biological coating 100 of Fig. 8. The biological coating 100 includes a surface layer 110 and an intermediate layer 120. The surface layer 110 is arranged on the intermediate layer 120 and at the outermost side of the biological coating 100. The surface layer 110 has a porosity greater than the intermediate layer 120. The intermediate layer 120 includes a plurality of second monomers connected in an unordered manner.

The connectivity between the plurality of first pores, between the plurality of second pores and among the plurality of first and second pores in the biological coating 100 affects the vascularization in the biological coating 100. In other words, low connectivity easily leads to the failure of interconnectivity between newly formed bones, resulting in insufficient of integration and continuity. Although the plurality of second monomers in the intermediate layer 120 connected in an ordered manner are able to ensure a good porosity and connectivity within the biological coating, such a structure of the biological coating is quite distinct from that of native cancellous bone. Native cancellous bone has a complicated structure with pores having a normally distributed pore size, pores of different sizes providing distinct biological functions. The structure formed by the first pores in the first monomers connected in an unordered manner and the second pores in the second monomers connected in an unordered manner is very similar to the native cancellous bone in natural three-dimensional structure and physiological functions, which thus enables to further improve microstructural and biomechanical properties of the biological coating 100.

The porosity variation is able to be achieved through diameter and/or density variation of first connecting rods 140 and second connecting rods. Porosities of the intermediate layer 120 and the surface layer 110 are uniform and invariable within each single layer and vary among different layers. Alternatively, porosities of the intermediate layer 120 and the surface layer 110 gradually decrease within each single layer along the direction from the outer side to the inner side. Alternatively, the entire biological coating has a porosity gradually decrease along the direction from the outer side to the inner side.

This embodiment also provides an implant. Referring to Fig. 10, a structural schematic of an implant according to embodiment 2 of present application, the plant includes a substrate layer 200 and the biological coating 100 according to embodiment 2. The intermediate layer 120 in the biological coating is disposed on the substrate layer 200, with the surface layer 110 arranged at the outermost side of the implant. The substrate layer 200 may also be a porous coating layer, which has a porosity lower than the intermediate layer 120.

The substrate layer 200 may be integrally formed by a 3D print. Alternatively, the entirety of the implant is integrally formed by a 3D print.

### Embodiment 3

This embodiment provides a biological coating 100. The biological coating 100 in this embodiment differs from the biological coating 100 in embodiment 1 in that the second monomers has a diamond structure.

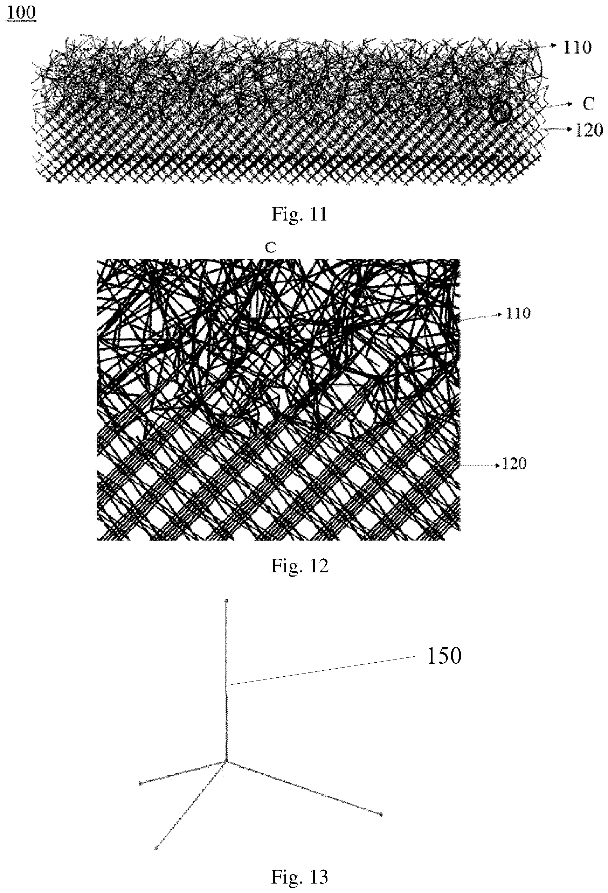

Reference is now made to Fig. 11, a structural schematic of the biological coating 100 according to embodiment 3 of present application, and to Fig. 12, a schematic enlarged view of portion C of the biological coating 100 of Fig. 11. The biological coating 100 includes a surface layer 110 and an intermediate layer 120. The surface layer 110 is disposed on the intermediate layer 120. The surface layer 110 is disposed at the outermost layer of the biological coating 100. The surface layer 110 has a porosity greater than the intermediate layer 120. The intermediate layer 120 includes a plurality of second monomers connected in an ordered manner. The second monomer a diamond structure constructed from a plurality of second connecting rods.

Referring to Fig. 13, a schematic diagram of a diamond structure according to embodiment 3 of the present application, the diamond structure is constructed from four second connecting rods 150 connected with one another. The four second connecting rods 150 are connected with one another at first ends thereof with second ends of the four second connecting rods 150 being separated from one another. The connected first ends of the four second connecting rods 150 are located at a center of a regular tetrahedron, and the second ends of the four second connecting rods 150 are located at four vertices of the regular tetrahedron respectively.

In this embodiment, the first monomer in the surface layer 110 has a structure of rhombic dodecahedron and the second monomer in the intermediate layer 120 has a diamond structure. As a result of the differently structured monomers in the surface and intermediate layers 110, 120, porosities and mechanical properties of the surface and intermediate layers 110, 120 are able to vary. Compared to the biological coating having varied porosities through merely varied diameters of the first connecting rods and the second connecting rods and/ or varied densities of arrangements for the first connecting rods and the second connecting rods, the biological coating 100 of this embodiment is able to avoid the easy breakage of the first or second connecting rods 140,150 arising from the excessively small diameter and the very sparse arrangement. In addition, the biological coating 100 of this embodiment is also able to avoid the problem of difficult powder removal arising from the excessively large diameter and the very dense arrangement of the connecting rods.

Preferably, in this embodiment, the porosity variation is also able to be achieved through the diameter and/or density variation of the first connecting rods 140 and the second connecting rods. In this embodiment, the influence on porosity by using different monomers in the surface and intermediate layers 110, 120 may be combined with the influence on porosity by diameter and/or density variation of the first connecting rods 140 and the second connecting rods 150, which enables to combine advantages of these two adjusting approaches for porosity, allowing achieving a higher stability and a better bone ingrowth performance of the biological coating 100.

Porosities of the intermediate layer 120 and the surface layer 110 are uniform and invariable within each single layer and vary among different layers. Alternatively, porosities of the intermediate layer 120 and the surface layer 110 gradually decrease within each single layer along the direction from the outer side to the inner side. Alternatively, the entire biological coating has a porosity gradually decrease along the direction from the outer side to the inner side.

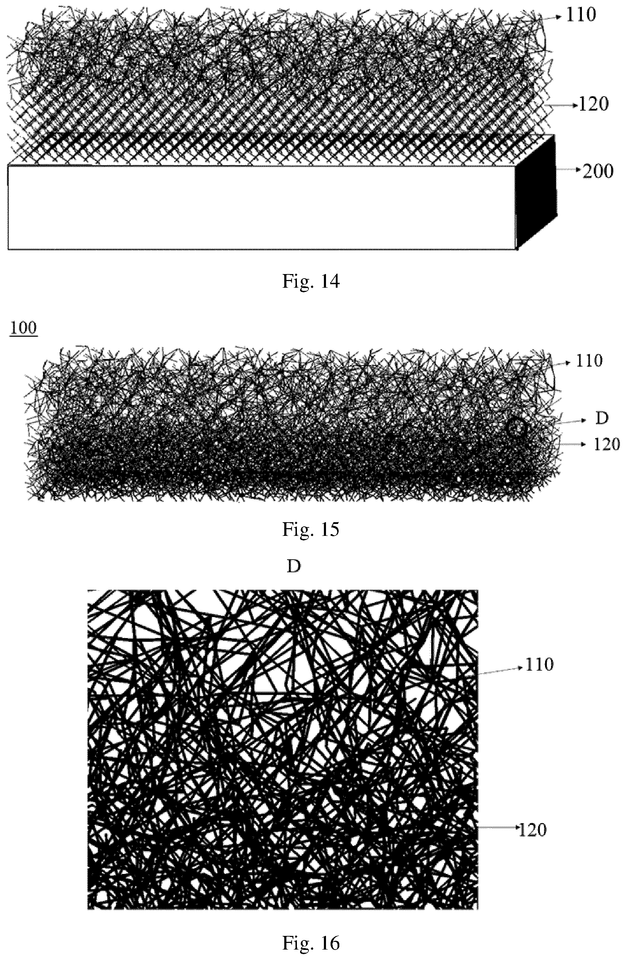

This embodiment also provides an implant. Referring to Fig. 14, a structural schematic of an implant according to embodiment 3 of present application, the plant includes a substrate layer 200 and the biological coating 100 according to embodiment 3. The intermediate layer 120 in the biological coating is disposed on the substrate layer 200, with the surface layer 110 arranged at the outermost side of the implant. The substrate layer 200 may also be a porous coating layer, which has a porosity lower than the intermediate layer 120.

The substrate layer 200 may be integrally formed by a 3D print. Alternatively, the entirety of the implant is integrally formed by a 3D print.

### Embodiment 4

This embodiment provides a biological coating 100. The biological coating 100 in this embodiment differs from the biological coating 100 in embodiment 1 in that the second monomers in the intermediate layer 120 are connected in an unordered manner, each monomer having a diamond structure.

Reference is now made to Fig. 15, a structural schematic of the biological coating 100 according to embodiment 4 of present application, and to Fig. 16, a schematic enlarged view of portion D of the biological coating 100 of Fig. 15. The biological coating 100 includes a surface layer 110 and an intermediate layer 120. The surface layer 110 is arranged on the intermediate layer 120 and at the outermost side of the biological coating 100. The surface layer 110 has a porosity greater than the intermediate layer 120. The intermediate layer 120 includes a plurality of second monomers connected in an unordered manner. The second monomer has a diamond structure constructed from a plurality of second connecting rods.

Similar to the embodiment 3, the diamond structure is constructed from four second connecting rods 150 connected with one another. The four second connecting rods are connected with one another at first ends thereof with second ends of the four second connecting rods being separated from one another. The connected first ends of the four second connecting rods are located at a center of a regular tetrahedron, and the second ends of the four second connecting rods 150 are located at four vertices of the regular tetrahedron respectively.

In this embodiment, the first monomer in the surface layer 110 has a structure of rhombic dodecahedron and the second monomer in the intermediate layer 120 has a structure of diamond. As a result of the differently structured monomers in the surface and intermediate layers 110, 120, porosities and mechanical properties of the surface and intermediate layers 110, 120 are able to vary. Compared to the biological coating 100 having varied porosities through merely varied diameters of the first connecting rods 140 and the second connecting rods and/ or varied densities of arrangements for the first connecting rods 140 and the second connecting rods, the biological coating 100 of this embodiment is able to avoid the easy breakage of the first or second connecting rods 140,150 arising from the excessively small diameter and the very sparse arrangement. In addition, the biological coating 100 of this embodiment is also able to avoid the problem of difficult powder removal arising from the excessively large diameter and the very dense arrangement of the connecting rods.

Preferably, in this embodiment, the porosity variation is also able to be achieved through the diameter and/or density variation of the first connecting rods 140 and the second connecting rods. In this embodiment, the influence on porosity by using different monomers in the surface and intermediate layers 110, 120 may be combined with the influence on porosity by diameter and/or density variation of the first connecting rods 140 and the second connecting rods, which enables to combine advantages of these two adjusting approaches for porosity, allowing achieving a higher stability and a better bone ingrowth performance of the biological coating 100.

In addition, native cancellous bone has a complicated structure with pores having a normally distributed pore size, pores of different sizes providing distinct biological functions. Therefore, the structure formed by the first pores in the first monomers connected in an unordered manner and the second pores in the second monomers connected in an unordered manner is very similar to the native cancellous bone in natural three-dimensional structure and physiological functions, which thus enables to further improve microstructural and biomechanical properties of the biological coating 100.

In addition, porosities of the intermediate layer 120 and the surface layer 110 are uniform and invariable within each single layer and vary among different layers. Alternatively, porosities of the intermediate layer 120 and the surface layer 110 gradually decrease within each single layer along the direction from the outer side to the inner side.

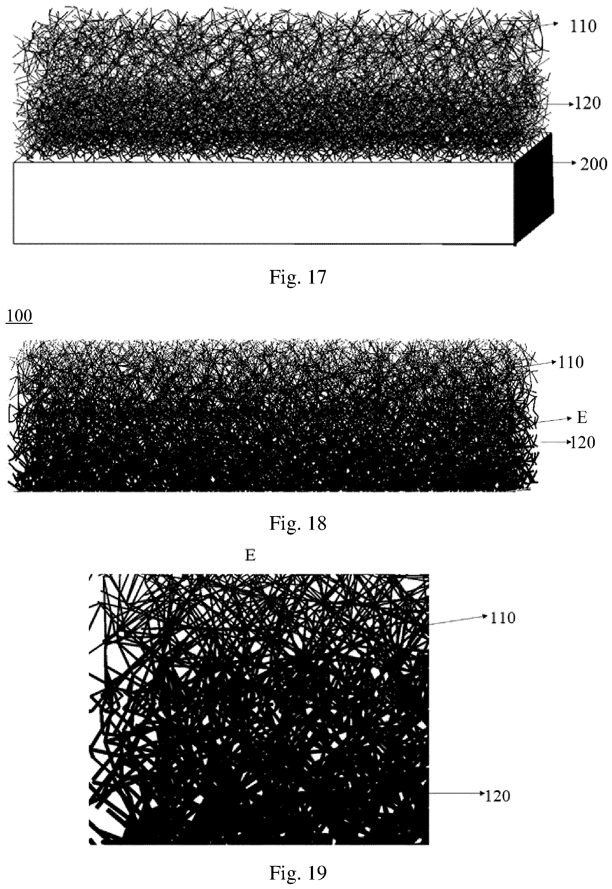

This embodiment also provides an implant. Referring to Fig. 17, a structural schematic of an implant according to embodiment 4 of present application, the plant includes a substrate layer 200 and the biological coating 100 according to embodiment 4. The intermediate layer 120 in the biological coating is disposed on the substrate layer 200, with the surface layer 110 arranged at the outermost side of the implant. The substrate layer 200 may also be a porous coating layer, which has a porosity lower than the intermediate layer 120.

The substrate layer 200 may be integrally formed by a 3D print. Alternatively, the entirety of the implant is integrally formed by a 3D print.

### Embodiment 5

This embodiment provides a biological coating 100. The biological coating 100 in this embodiment differs from the biological coating 100 in embodiment 1 in that the second monomers in the intermediate layer 120 are connected in an unordered manner, each monomer having a diamond structure and the entire biological coating 100 has a porosity continuously changing in gradient from the outer side to the inner side.

Reference is now made to Fig. 18, a structural schematic of the biological coating 100 according to embodiment 5 of present application, and to Fig. 19, a schematic enlarged view of portion E of the biological coating 100 of Fig. 18. The biological coating 100 includes a surface layer 110 and an intermediate layer 120. The surface layer 110 is arranged on the intermediate layer 120 and at the outermost side of the biological coating 100. The surface layer 110 has a porosity greater than the intermediate layer 120. The intermediate layer 120 includes a plurality of second monomers connected in an unordered manner. The second monomer has a diamond structure constructed from a plurality of second connecting rods.

Similar to the embodiment 4, the diamond structure is constructed from four second connecting rods 150 connected with one another. The four second connecting rods are connected with one another at first ends thereof with second ends of the four second connecting rods being separated from one another. The connected first ends of the four second connecting rods are located at a center of a regular tetrahedron, and the second ends of the four second connecting rods 150 are located at four vertices of the regular tetrahedron respectively.

In this embodiment, the first monomer in the surface layer 110 has a structure of rhombic dodecahedron and the second monomer in the intermediate layer 120 has a structure of diamond. As a result of the differently structured monomers in the surface and intermediate layers 110, 120, porosities and mechanical properties of the surface and intermediate layers 110, 120 are able to vary. Compared to the biological coating 100 having varied porosities through merely varied diameters of the first connecting rods 140 and the second connecting rods and/ or varied densities of arrangements for the first connecting rods 140 and the second connecting rods, the biological coating 100 of this embodiment is able to avoid the easy breakage of the first or second connecting rods 140,150 arising from the excessively small diameter and the very sparse arrangement. In addition, the biological coating 100 of this embodiment is also able to avoid the problem of difficult powder removal arising from the excessively large diameter and the very dense arrangement of the connecting rods.

Preferably, in this embodiment, the porosity variation is also able to be achieved through the diameter and/or density variation of the first connecting rods 140 and the second connecting rods. In this embodiment, the influence on porosity by using different monomers in the surface and intermediate layers 110, 120 may be combined with the influence on porosity by diameter and/or density variation of the first connecting rods 140 and the second connecting rods, which enables to combine advantages of these two adjusting approaches for porosity, allowing achieving a higher stability and a better bone ingrowth performance of the biological coating 100.

In addition, native cancellous bone has a complicated structure with pores having a normally distributed pore size, pores of different sizes providing distinct biological functions. Therefore, the structure formed by the first pores in the first monomers connected in an unordered manner and the second pores in the second monomers connected in an unordered manner is very similar to the native cancellous bone in natural three-dimensional structure and physiological functions, which thus enables to further improve microstructural and biomechanical properties of the biological coating 100.

The intermediate layer 120 has a porosity lower than the surface layer 110, and the entire biological coating 100 has a porosity gradually decreasing from the outer side to the inner side. In other words, porosities of the intermediate layer 120 and the surface layer 110 continuously vary in gradient along the direction from the outer side to the inner side, which makes fracture between layers arising from discontinuous in properties (e.g., porosity, rod diameter, density, etc.) less prone. This results in even better mechanical properties for the intermediate layer 120, a good bone ingrowth performance of the surface layer 110. Moreover, as a good transition is presented among the surface layer 110 and the intermediate layer 120, the biological coating is able to have a better stability.

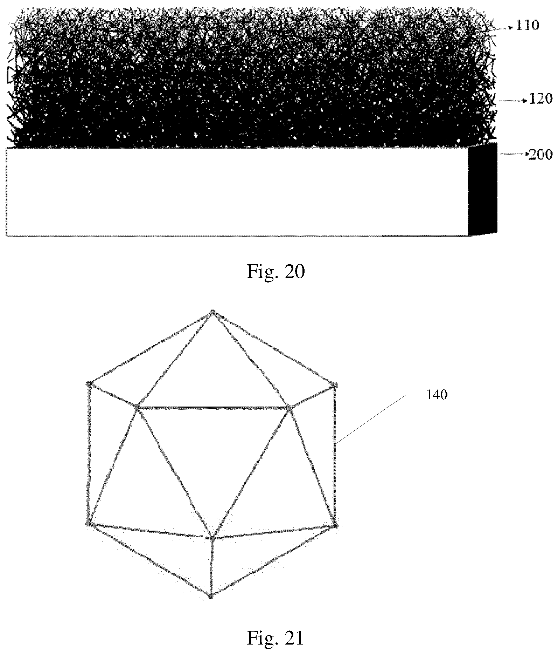

This embodiment also provides an implant. Referring to Fig. 20, a structural schematic of an implant according to embodiment 5 of present application, the plant includes a substrate layer 200 and the biological coating 100 according to embodiment 5. The intermediate layer 120 in the biological coating is disposed on the substrate layer 200, with the surface layer 110 arranged at the outermost side of the implant. The substrate layer 200 may also be a porous coating layer, which has a porosity lower than the intermediate layer 120.

The substrate layer 200 may be integrally formed by a 3D print. Alternatively, the entirety of the implant is integrally formed by a 3D print.

In each of the embodiments 3, 4 and 5, the second monomers in the intermediate layer has a diamond structure, where the plurality of diamond structures connected in an ordered manner in embodiment 3 while the plurality of diamond structures connected in an unordered manner in embodiments 4 and 5. For the diamond structures connected in an ordered manner, it is formed from four second connecting rods connected with one another. The four second connecting rods 150 are connected with one another at first ends thereof with second ends of the four second connecting rods 150 being separated from one another. The connected first ends of the four second connecting rods 150 are located at a center of a regular tetrahedron, and the second ends of the four second connecting rods 150 are located at four vertices of the regular tetrahedron respectively. For the diamond structures connected in an unordered manner, angles between second connecting rods changes and the length of the second connecting rods also changes. That is to say, the diamond structures connected in an unordered manner is actually not a regular tetrahedral structure, but a deformed tetrahedral structure.

The regular tetrahedron has four faces each being a triangle. Such structure is very stable to provide excellent mechanical properties. Additionally, the tetrahedral structure has only four connected second connecting rods at each vertex, while four second connecting rods is the minimum number of rods required to stabilize a three-dimensional structure in space. Therefore, in the same space, such structure enables pores between the four second connecting rods to be large while allowing achieving a good stability. When adjusting porosity by adjustment of diameters of the second connecting rod, this offers a good porosity to the intermediate layer while enabling to avoid the easy breakage of rods arising from the excessively small diameter and the very sparse arrangement, or this offers good mechanical properties to the intermediate layer while enabling to avoid the problem of difficult powder removal arising from the excessively large diameter and the very dense arrangement of the connecting rods. Thus, it is most preferred that the second monomer in the intermediate layer has a diamond structure. However, in other embodiments, the second monomer in the intermediate layer may be otherwise structured, and the present application is not limited this.

### Embodiment 6

This embodiment provides a biological coating 100. The biological coating 100 includes a surface layer 110 and an intermediate layer 120. The surface layer 110 is arranged on the intermediate layer 120 and at the outermost side of the biological coating 100. The surface layer 110 has a porosity greater than the intermediate layer 120.

The surface layer 110 includes a plurality of first monomers connected in an unordered manner. A plurality of first pores are formed between the plurality of first monomers and within the interior of the plurality of first monomers.

The first monomers may have an N-hedron structure, where N>_ 10. For example, the first monomers may be one selected from the group consisting of rhombic dodecahedron, icosahedron and icosidodecahedron (Fullerene). The icosahedral structure can refer to Fig. 21 which schematically shows an icosahedral structure according to embodiment 6 of present application.

The intermediate layer 120 includes a plurality of second monomers connected in an ordered manner. A plurality of second pores are formed between the plurality of second monomers and within the interior of the plurality of second monomers.

The second monomers may have an M-hedron structure, where N<10. For example, the second monomer may have a structure selected from the group consisting of tetrahedron, cube and octahedron.

The second monomer may also have a structure of diamond structure or cellular structure.

In other embodiments, the second monomers may be arranged in an unordered manner.

This embodiment also provides an implant including a substrate layer 200 and the biological coating 100 according to embodiment 6. The intermediate layer 120 in the biological coating is disposed on the substrate layer 200, with the surface layer 110 arranged at the outermost side of the implant. The substrate layer 200 may also be a porous coating layer, which has a porosity lower than the intermediate layer 120.

The substrate layer 200 may be integrally formed by a 3D print. Alternatively, the entirety of the implant is integrally formed by a 3D print.

### Embodiment 7

This embodiment provides a biological coating 100 including only a surface layer 110.

The surface layer 110 includes a plurality of first monomers connected in an unordered manner. A plurality of first pores are formed between the plurality of first monomers and within the interior of the plurality of first monomers.

The first monomers may have a K-hedron structure, where K≥4. For example, the first monomers may be one selected from the group consisting of rhombic dodecahedron, icosahedron, icosidodecahedron (Fullerene), tetrahedron, cube and octahedron.

The first monomer may also have a structure of diamond structure or cellular structure.

This embodiment also provides an implant including a substrate layer 200 and the biological coating 100 according to embodiment 7. The substrate layer 200 may also be a porous coating layer, which has a porosity lower than the surface layer 110.

The substrate layer 200 may be integrally formed by a 3D print. Alternatively, the entirety of the implant is integrally formed by a 3D print.

In above embodiments, the surface layer 110 is disposed on an outermost side of the biological coating 100 and includes a plurality of first monomers connected in an unordered manner, a plurality of first pores formed between the plurality of first monomers and within the interior of the plurality of first monomers. That is, the unordered connections between the first monomers in the surface layer 110 allows the plurality of first pores in the surface layer 110 being in an unordered arrangement, which facilitates cells of corresponding bone tissues to grow into the surface layer 110, thus allowing an improved bone ingrowth performance and a long-term stability.

In above embodiments, the biological coating 100 further includes at least one intermediate layer 120, and the surface layer 110 and the at least one intermediate layer 120 are arranged in sequence along a direction from an outer side of the biological coating to an inner side of the biological coating, the porosity of the biological coating 100 decreasing gradually along the direction from the outer side of the biological coating 100 to the inner side of the biological coating 100. In this way, the surface layer 110 of the biological coating is able to own a good bone ingrowth performance and a long-term stability, and the intermediate layer 120 of the biological coating 100 is able to own a good short-term stability, which enables to offer an overall stability to the biological coating 100.

In above embodiments, due to the ordered connection between the plurality of second monomers in the intermediate layer 120, the plurality of second pores in the intermediate layer 120 are able to be in the ordered arrangement, which offers a good mechanical property to the intermediate layer 120 and biological coating 100.

In above embodiments, both the first and second monomers, and hence both the first and second pores therein, are interconnected in a disorderly manner. This closely emulates the natural three-dimensional and physiological characteristics of native cancellous bone, allowing the biological coating 100 to have further improved microstructural and biomechanical properties.

In above embodiments, since each of the first and second monomers connected in the unordered manner, the first pores in the first monomers connected in the unordered manner and the second pores in the second monomers connected in the unordered manner form a structure that is very similar to native cancellous bone in natural three-dimensional structure and physiology functions, which allows to further improve microstructure and biomechanical properties of the biological coating 100.

In above embodiments, as the first monomer in the surface layer 110 has a different structure with respect to the second monomers in the intermediate layer 120, the surface layer 110 has a different porosity and mechanical properties with respect to the intermediate layer 120. Compared to the biological coating 100 having varied porosities through merely varied diameters of the first connecting rods 140 in the first monomers and the second connecting rods 150 in the second monomers and/ or varied densities of arrangements for the first connecting rods 140 in the first monomers and the second connecting rods 150 in the second monomers, such embodiment is able to avoid the easy breakage of the first or second connecting rods 140, 150 arising from the excessively small diameter and the very sparse arrangement. In addition, such embodiment is also able to avoid the problem of difficult powder removal arising from the excessively large diameter and the very dense arrangement of the first and second connecting rods 140, 150.

In above embodiments, porosities of the surface 110 and intermediate 120 layers continuously vary in gradient along the direction from the outer side of the biological coating 100 to the inner side of the biological coating 100, which makes fracture between layers arising from discontinuous in properties (e.g., porosity, rod diameter, density, etc.) less prone. This results in even better mechanical properties for the intermediate layer 120, a good bone ingrowth performance of the surface layer 110. Moreover, as a good transition is presented among the surface layer 110 and the intermediate layers 120, the biological coating is able to have a better stability.

In above embodiments, the biological coating may be integrally formed by a 3D print. This allows the porosities of the surface and intermediate layers 110, 120 to vary in a controllable manner, resulting in a good interconnectivity between the first pores, between the second pores and among the first and second pores in the biological coating 100.

The description presented above is merely that of a few preferred embodiments of the present application and is not intended to limit the scope of present application in any sense. Any and all changes and modifications made by those of ordinary skill in the art based on the above disclosure fall within the protection scope of the appended claims.

## Claims

1. A biological coating (100) comprising a surface layer (110), **characterized in that**, the surface layer (110) comprises a plurality of first monomers connected in an unordered manner, and a plurality of first pores are formed between the plurality of first monomers and within an interior of the plurality of first monomers, **characterized in that** the biological coating (100) further comprises at least one intermediate layer (120), wherein the surface layer (110) is disposed on an outermost side of the biological coating, wherein the surface layer (110) and the at least one intermediate layer (120) are arranged along a direction from an outer side of the biological coating to an inner side of the biological coating, and wherein a porosity of the biological coating decreases gradually along the direction from the outer side of the biological coating to the inner side of the biological coating, and **in that** the at least one intermediate layer (120) comprises a plurality of second monomers connected in an ordered manner, and a plurality of second pores are formed between the plurality of second monomers and within the interior of the plurality of second monomers.

2. The biological coating (100) of claim 1, wherein the first monomer in the surface layer (110) has a different structure with respect to the second monomer in the at least one intermediate layer (120).

3. The biological coating (100) of claim 2, wherein the first monomer has anN-hedron structure, where N ≥ 10, and wherein the second monomer has an M-hedron structure, where M<10.

4. The biological coating (100) of claim 2, wherein a structure of the first monomer is one selected from the group consisting of rhombic dodecahedron, icosahedron and icosidodecahedron, and wherein a structure of the second monomer is one selected from the group consisting of diamond structure, cellular structure, tetrahedron, cube and octahedron.

5. The biological coating (100) of claim 2, wherein the second monomer has a diamond structure formed from four second connecting rods (150) connected with one another, wherein the four second connecting rods are connected with one another at first ends thereof with second ends of the four second connecting rods (150) being separated from one another, wherein the connected first ends of the four second connecting rods are located at a center of a regular tetrahedron, and the second ends of the four second connecting rods (150) are located at four vertices of the regular tetrahedron respectively.

6. The biological coating (100) of claim 1, wherein the at least one intermediate layer (120) has at least two intermediate layers, wherein different types of second monomers are provided.

7. The biological coating (100) of claim 1, wherein porosities of the intermediate layer(s) (120) and the surface layer (110) are uniform and invariable within each single layer and gradually decrease among different layers along the direction from the outer side of the biological coating (100) to the inner side of the biological coating; or porosities of the intermediate layer(s) (120) and the surface layer (110) gradually decrease within each single layer along the direction from the outer side of the biological coating (100) to the inner side of the biological coating and gradually decrease among different layers along the direction from the outer side of the biological coating (100) to the inner side of the biological coating; or the biological coating has a porosity continuously changing in gradient along the direction from the outer side of the biological coating (100) to the inner side of the biological coating.

8. The biological coating (100) of claim 1, wherein the first monomer is constructed from a plurality of first connecting rods (140)connected to one another, and wherein the second monomer is constructed from a plurality of second connecting rods (150) connected to one another, wherein the first connecting rods (140) are smaller than the second connecting rods (150) in diameter, the first connecting rods (140) in the surface layer (110) having an identical diameter, the second connecting rods (150) in the intermediate layer(s) (120) having an identical diameter; and/or wherein the first connecting rods (140) are arranged in the surface layer with a smaller density compared to the second connecting rods (150) in the intermediate layer(s), the first connecting rods (140) in the surface layer (110) being arranged with uniform density, the second connecting rods (150) in the intermediate layer(s) (120) being arranged with uniform density; or
wherein the first connecting rods (140) in the surface layer (110) have a diameter gradually increasing along the direction from the outer side of the biological coating (100) to the inner side of the biological coating, the second connecting rods (150) in the intermediate layer(s) (120) having a diameter gradually increasing along the direction from the outer side of the biological coating (100) to the inner side of the biological coating, the first connecting rods (140) in the surface layer (110) being smaller than the second connecting rods in the intermediate layer(s) (120) in diameter; and/or wherein the first connecting rods in the surface layer are arranged in a density gradually increasing along the direction from the outer side of the biological coating (100) to the inner side of the biological coating, the second connecting rods (150) in the intermediate layer(s) (120) are arranged in a density gradually increasing along the direction from the outer side of the biological coating to the inner side of the biological coating, and the first connecting rods (140) in the surface layer (110) are arranged in a smaller density compared to the second connecting rods (150) in the intermediate layer(s) (120); or
wherein the biological coating (100) has diameters of the first connecting rods (140) and the second connecting rods (150) gradually increasing along the direction from the outer side of the biological coating to the inner side of the biological coating, and/or the biological coating has the first connecting rods (140) and the second connecting rods (150) arranged in a density gradually increasing along the direction from the outer side of the biological coating to the inner side of the biological coating.

9. The biological coating (100) of any one of claims 1 to 8 designed with a computer software and integrally formed by a 3D print.

10. An implant comprising a substrate layer (200) and the biological coating (100) of any one of claims 1 to 8, wherein the biological coating is disposed on the substrate layer (200), and the surface layer (110) of the biological coating is disposed on an outermost side of the implant.

11. The implant of claim 10, wherein an entirety of the implant is integrally formed by a 3D print.

## Patentansprüche

1. Biologische Beschichtung (100), umfassend eine Oberflächenschicht (110), **dadurch gekennzeichnet, dass** die Oberflächenschicht (110) eine Vielzahl von ersten Monomeren umfasst, die auf eine ungeordnete Weise verbunden sind, und eine Vielzahl von ersten Poren zwischen der Vielzahl von ersten Monomeren und innerhalb eines Inneren der Vielzahl von ersten Monomeren gebildet sind, **dadurch gekennzeichnet, dass** die biologische Beschichtung (100) ferner mindestens eine Zwischenschicht (120) umfasst, wobei die Oberflächenschicht (110) auf einer äußersten Seite der biologischen Beschichtung angeordnet ist, wobei die Oberflächenschicht (110) und die mindestens eine Zwischenschicht (120) entlang einer Richtung von einer Außenseite der biologischen Beschichtung zu einer Innenseite der biologischen Beschichtung angeordnet sind, und wobei eine Porosität der biologischen Beschichtung entlang der Richtung von der Außenseite der biologischen Beschichtung zu der Innenseite der biologischen Beschichtung allmählich abnimmt, und dass die mindestens eine Zwischenschicht (120) eine Vielzahl von zweiten Monomeren umfasst, die auf eine geordnete Weise verbunden sind, und eine Vielzahl von zweiten Poren zwischen der Vielzahl von zweiten Monomeren und innerhalb des Inneren der Vielzahl von zweiten Monomeren gebildet sind.

2. Biologische Beschichtung (100) nach Anspruch 1, wobei das erste Monomer in der Oberflächenschicht (110) eine andere Struktur als das zweite Monomer in der mindestens einen Zwischenschicht (120) aufweist.

3. Biologische Beschichtung (100) nach Anspruch 2, wobei das erste Monomer eine N-Hedron-Struktur aufweist, wobei N ≥ 10, und wobei das zweite Monomer eine M-Hedron-Struktur aufweist, wobei M<10.

4. Biologische Beschichtung (100) nach Anspruch 2, wobei eine Struktur des ersten Monomers eine ist, ausgewählt aus der Gruppe, bestehend aus Rhombendodekaeder, Ikosaeder und Ikosidodekaeder, und wobei eine Struktur des zweiten Monomers eine ist, ausgewählt aus der Gruppe, bestehend aus Diamantstruktur, zellulärer Struktur, Tetraeder, Würfel und Oktaeder.

5. Biologische Beschichtung (100) nach Anspruch 2, wobei das zweite Monomer eine Diamantstruktur aufweist, die aus vier zweiten Verbindungsstäben (150) gebildet ist, die miteinander verbunden sind, wobei die vier zweiten Verbindungsstäbe an ihren ersten Enden miteinander verbunden sind, wobei die zweiten Enden der vier zweiten Verbindungsstäbe (150) voneinander getrennt sind, wobei die verbundenen ersten Enden der vier zweiten Verbindungsstäbe in einer Mitte eines regelmäßigen Tetraeders angeordnet sind und die zweiten Enden der vier zweiten Verbindungsstäbe (150) jeweils an vier Spitzen des regelmäßigen Tetraeders angeordnet sind.

6. Biologische Beschichtung (100) nach Anspruch 1, wobei die mindestens eine Zwischenschicht (120) mindestens zwei Zwischenschichten aufweist, wobei verschiedene Arten von zweiten Monomeren bereitgestellt sind.

7. Biologische Beschichtung (100) nach Anspruch 1, wobei die Porositäten der Zwischenschicht(en) (120) und der Oberflächenschicht (110) innerhalb jeder einzelnen Schicht gleichförmig und unveränderlich sind und unter den verschiedenen Schichten entlang der Richtung von der Außenseite der biologischen Beschichtung (100) zu der Innenseite der biologischen Beschichtung allmählich abnehmen; oder die Porositäten der Zwischenschicht(en) (120) und der Oberflächenschicht (110) innerhalb jeder einzelnen Schicht entlang der Richtung von der Außenseite der biologischen Beschichtung (100) zu der Innenseite der biologischen Beschichtung allmählich abnehmen und zwischen verschiedenen Schichten entlang der Richtung von der Außenseite der biologischen Beschichtung (100) zu der Innenseite der biologischen Beschichtung allmählich abnehmen; oder die biologische Beschichtung eine Porosität aufweist, die sich entlang der Richtung von der Außenseite der biologischen Beschichtung (100) zu der Innenseite der biologischen Beschichtung kontinuierlich in dem Gradienten ändert.

8. Biologische Beschichtung (100) nach Anspruch 1, wobei das erste Monomer aus einer Vielzahl von miteinander verbundenen ersten Verbindungsstäben (140) aufgebaut ist, und wobei das zweite Monomer aus einer Vielzahl von miteinander verbundenen zweiten Verbindungsstäben (150) aufgebaut ist,
wobei die ersten Verbindungsstäbe (140) im Durchmesser kleiner sind als die zweiten Verbindungsstäbe (150), wobei die ersten Verbindungsstäbe (140) in der Oberflächenschicht (110) einen gleichen Durchmesser aufweisen, wobei die zweiten Verbindungsstäbe (150) in der/den Zwischenschicht(en) (120) einen gleichen Durchmesser aufweisen; und/oder wobei die ersten Verbindungsstäbe (140) in der Oberflächenschicht mit einer geringeren Dichte angeordnet sind als die zweiten Verbindungsstäbe (150) in der/den Zwischenschicht(en), wobei die ersten Verbindungsstäbe (140) in der Oberflächenschicht (110) mit gleichförmiger Dichte angeordnet sind, wobei die zweiten Verbindungsstäbe (150) in der/den Zwischenschicht(en) (120) mit gleichförmiger Dichte angeordnet sind; oder
wobei die ersten Verbindungsstäbe (140) in der Oberflächenschicht (110) einen Durchmesser aufweisen, der entlang der Richtung von der Außenseite der biologischen Beschichtung (100) zu der Innenseite der biologischen Beschichtung allmählich zunimmt, wobei die zweiten Verbindungsstäbe (150) in der/den Zwischenschicht(en) (120) einen Durchmesser aufweisen, der entlang der Richtung von der Außenseite der biologischen Beschichtung (100) zu der Innenseite der biologischen Beschichtung allmählich zunimmt, wobei die ersten Verbindungsstäbe (140) in der Oberflächenschicht (110) einen kleineren Durchmesser als die zweiten Verbindungsstäbe in der/den Zwischenschicht(en) (120) aufweisen;
und/oder wobei die ersten Verbindungsstäbe in der Oberflächenschicht in einer Dichte angeordnet sind, die entlang der Richtung von der Außenseite der biologischen Beschichtung (100) zu der Innenseite der biologischen Beschichtung allmählich zunimmt, die zweiten Verbindungsstäbe (150) in der/den Zwischenschicht(en) (120) in einer Dichte angeordnet sind, die entlang der Richtung von der Außenseite der biologischen Beschichtung zu der Innenseite der biologischen Beschichtung allmählich zunimmt, und die ersten Verbindungsstäbe (140) in der Oberflächenschicht (110) in einer geringeren Dichte im Vergleich zu den zweiten Verbindungsstäben (150) in der/den Zwischenschicht(en) (120) angeordnet sind; oder
wobei die biologische Beschichtung (100) Durchmesser der ersten Verbindungsstäbe (140) und der zweiten Verbindungsstäbe (150) aufweist, die entlang der Richtung von der Außenseite der biologischen Beschichtung zu der Innenseite der biologischen Beschichtung allmählich zunehmen, und/oder die biologische Beschichtung die ersten Verbindungsstäbe (140) und die zweiten Verbindungsstäbe (150) aufweist, die in einer Dichte angeordnet sind, die entlang der Richtung von der Außenseite der biologischen Beschichtung zu der Innenseite der biologischen Beschichtung allmählich zunimmt.

9. Biologische Beschichtung (100) nach einem der Ansprüche 1 bis 8, konstruiert mit einer Computersoftware und ganzheitlich gebildet durch einen 3D-Druck.

10. Implantat, umfassend eine Substratschicht (200) und die biologische Beschichtung (100) nach einem der Ansprüche 1 bis 8, wobei die biologische Beschichtung auf der Substratschicht (200) angeordnet ist und die Oberflächenschicht (110) der biologischen Beschichtung auf einer äußersten Seite des Implantats angeordnet ist.

11. Implantat nach Anspruch 10, wobei die Gesamtheit des Implantats durch einen 3D-Druck gebildet wird.

## Revendications

1. Revêtement biologique (100) comprenant une couche de surface (110), **caractérisé en ce que** la couche de surface (110) comprend une pluralité de premiers monomères reliés de manière non ordonnée, et une pluralité de premiers pores sont formés entre la pluralité de premiers monomères et à l'intérieur de la pluralité de premiers monomères, **caractérisé en ce que** le revêtement biologique (100) comprend en outre au moins une couche intermédiaire (120), dans lequel la couche de surface (110) est disposée sur un côté le plus à l'extérieur du revêtement biologique, dans lequel la couche de surface (110) et au moins une couche intermédiaire (120) sont disposées le long d'une direction allant d'un côté extérieur du revêtement biologique vers un côté intérieur du revêtement biologique, et dans lequel une porosité du revêtement biologique diminue progressivement le long de la direction allant du côté extérieur du revêtement biologique vers le côté intérieur du revêtement biologique, et **en ce que** la au moins une couche intermédiaire (120) comprend une pluralité de seconds monomères reliés de manière ordonnée, et une pluralité de seconds pores sont formés entre la pluralité de seconds monomères et à l'intérieur de la pluralité de seconds monomères.

2. Revêtement biologique (100) selon la revendication 1, dans lequel le premier monomère dans la couche de surface (110) a une structure différente au second monomère dans l'au moins une couche intermédiaire (120).

3. Revêtement biologique (100) selon la revendication 2, dans lequel le premier monomère a une structure N-èdre, où N ≥ 10, et dans lequel le second monomère a une structure M-èdre, où M<10.

4. Revêtement biologique (100) selon la revendication 2, dans lequel la structure du premier monomère est l'une choisie dans le groupe constitué par les dodécaèdre, icosaèdre et icosidodécaèdre rhombiques, et dans lequel la structure du second monomère est l'une choisie dans le groupe constitué par la structure en losange, la structure cellulaire, le tétraèdre, le cube et l'octaèdre.

5. Revêtement biologique (100) selon la revendication 2, dans lequel le second monomère a une structure en losange formée de quatre secondes tiges de liaison (150) reliées les unes aux autres, dans lequel les quatre secondes tiges de liaison sont reliées les unes aux autres à leurs premières extrémités, les secondes extrémités des quatre secondes tiges de liaison (150) étant séparées les unes des autres, dans lequel les premières extrémités reliées des quatre secondes tiges de liaison sont situées au centre d'un tétraèdre régulier, et les secondes extrémités des quatre secondes tiges de liaison (150) sont situées respectivement au niveau des quatre sommets du tétraèdre régulier.

6. Revêtement biologique (100) selon la revendication 1, dans lequel l'au moins une couche intermédiaire (120) comporte au moins deux couches intermédiaires, dans lequel différents types de seconds monomères sont fournis.

7. Revêtement biologique (100) selon la revendication 1, dans lequel les porosités de la ou des couches intermédiaires (120) et de la couche de surface (110) sont uniformes et invariables à l'intérieur de chaque couche individuelle et diminuent progressivement entre les différentes couches le long de la direction allant de la face externe du revêtement biologique (100) vers la face interne du revêtement biologique ; ou les porosités de la ou des couches intermédiaires (120) et de la couche de surface (110) diminuent progressivement à l'intérieur de chaque couche individuelle le long de la direction allant de la face externe du revêtement biologique (100) vers la face interne du revêtement biologique et diminuent progressivement entre les différentes couches le long de la direction allant de la face externe du revêtement biologique (100) vers la face interne du revêtement biologique ; ou le revêtement biologique a une porosité qui change continuellement de gradient le long de la direction allant de la face externe du revêtement biologique (100) vers la face interne du revêtement biologique.

8. Revêtement biologique (100) selon la revendication 1, dans lequel le premier monomère est construit à partir d'une pluralité de premières tiges de liaison (140) reliées les unes aux autres, et dans lequel le second monomère est construit à partir d'une pluralité de secondes tiges de liaison (150) reliées les unes aux autres,
dans lequel les premières tiges de liaison (140) sont plus petites que les secondes tiges de liaison (150) en termes de diamètre, les premières tiges de liaison (140) dans la couche de surface (110) ayant un diamètre identique, les secondes tiges de liaison (150) dans la ou les couches intermédiaires (120) ayant un diamètre identique ; et/ou dans lequel les premières tiges de liaison (140) sont disposées dans la couche de surface avec une densité inférieure à celle des secondes tiges de liaison (150) dans la ou les couches intermédiaires, les premières tiges de liaison (140) dans la couche de surface (110) étant disposées avec une densité uniforme, les secondes tiges de liaison (150) dans la ou les couches intermédiaires (120) étant disposées avec une densité uniforme ; ou
dans lequel les premières tiges de liaison (140) dans la couche de surface (110) ont un diamètre qui augmente progressivement dans la direction allant de la face externe du revêtement biologique (100) vers la face interne du revêtement biologique, les secondes tiges de liaison (150) dans la ou les couches intermédiaires (120) ayant un diamètre qui augmente progressivement dans la direction allant de la face externe du revêtement biologique (100) vers la face interne du revêtement biologique, les premières tiges de liaison (140) dans la couche de surface (110) étant plus petites que les secondes tiges de liaison dans la ou des couches intermédiaires (120) en termes de diamètre ;
et/ou dans lequel les premières tiges de liaison dans la couche de surface sont disposées dans une densité qui augmente progressivement dans la direction allant de la face externe du revêtement biologique (100) vers la face interne du revêtement biologique, les secondes tiges de liaison (150) dans la ou les couches intermédiaires (120) sont disposées dans une densité qui augmente progressivement dans la direction allant de la face externe du revêtement biologique vers la face interne du revêtement biologique, et les premières tiges de liaison (140) dans la couche de surface (110) sont disposées dans une densité inférieure à celle des secondes tiges de liaison (150) dans la ou les couches intermédiaires (120) ; ou
dans lequel le revêtement biologique (100) présente des diamètres des premières tiges de liaison (140) et des secondes tiges de liaison (150) qui augmentent progressivement dans la direction allant de la face externe du revêtement biologique vers la face interne du revêtement biologique, et/ou le revêtement biologique présente les premières tiges de liaison (140) et les secondes tiges de liaison (150) disposées selon une densité qui augmente progressivement dans la direction allant de la face externe du revêtement biologique vers la face interne du revêtement biologique.

9. Revêtement biologique (100) selon l'une quelconque des revendications 1 à 8 conçu à l'aide d'un logiciel informatique et formé intégralement par impression 3D.

10. Implant comprenant une couche de substrat (200) et le revêtement biologique (100) selon l'une quelconque des revendications 1 à 8, dans lequel le revêtement biologique est disposé sur la couche de substrat (200), et la couche de surface (110) du revêtement biologique est disposée sur un côté le plus à l'extérieur de l'implant.

11. Implant selon la revendication 10, dans lequel l'ensemble de l'implant est formé intégralement par une impression 3D.
